# EUROPÄISCHE PATENTSCHRIFT

(11) **EP 1 462 073 B1**
(45) Veröffentlichungstag und Bekanntmachung des Hinweises auf die Patenterteilung: **03.12.2008**
(21) Anmeldenummer: 04090098.7
(22) Anmeldetag: 10.03.2004
(51) Int. Cl.: A61F 7/00

(54) **Wärme/Kältevorrichtung**
Heating and cooling device
Dispositif de chauffage et de refroidissement

(30) Priorität: 25.03.2003 DE 10314138
(43) Veröffentlichungstag der Anmeldung: 29.09.2004
(73) Patentinhaber: Krüger & Gothe GmbH, 39418 Stassfurt (DE)
(72) Erfinder: Krüger, Bernd, 06429 Nienburg (DE)
(74) Vertreter: Theobald, Andreas

(56) Entgegenhaltungen:
- US-A- 4 585 002
- US-A- 4 741 338
- US-A1- 2002 107 543

## Beschreibung

Die vorliegende Erfindung bezieht sich auf eine Wärme/Kältevorrichtung, insbesondere auf eine Wärme/Kältevorrichtung für ein Schmerzbehandlungsgerät, mit einer Heizeinrichtung und einer Kühleinrichtung zum wechselwiesen Erwärmen und Kühlen eines Heiz/Kühlelementes. Das Dokument US-A-2002/0107543 stellt der nädute Stand der Technik dar.

Häufig werden Schmerzen, wie etwa Migräne und Spannungskopfschmerzen, sowie Tinitusbeschwerden und vergleichbare Beschwerden medikamentös behandelt. In den vergangenen Jahren wurden jedoch auch alternative Ansätze der Schmerzbehandlung entwickelt. Einige zielen darauf ab, Schmerzen mit Hilfe einer Wärme/Kältebehandlung zu beseitigen oder zumindest zu verringern.

Ein Verfahren und ein Gerät zum Durchführen einer Wärme/Kältebehandlung sind in DE 100 65 592 beschrieben.

Das in DE 100 65 592 beschriebe Verfahren beruht darauf, dem Patienten im rhythmischen Wechsel Wärme und Kälte zuzuführen. Je nach Art der Schmerzen erfolgt die Zufuhr von Wärme bzw. Kälte über jeweils gleiche oder über unterschiedliche Zeitabschnitte. Die Wärme bzw. Kälte wirkt über eine Zeitdauer von Sekunden oder Minuten ein, bevor von Wärme auf Kälte oder von Kälte auf Wärme umgeschaltet wird.

Das Gerät, mit dem diese Schmerzbehandlung erfolgt, weist einen Kopfhörer mit zwei Muscheln auf. In einer Ausführungsform sind in der Muschel ein Wärmeelement und ein Kälteelement nebeneinander angeordnet. Gesteuert von einer Zeitintervallsteuerung erfolgt ein wechselweises Aufbringen des Wärme- und des Kälteelementes auf die Haut. Dies führt jedoch dazu, dass die Wärme und die Kälte an verschiedenen Hautstellen aufgebracht wird. In einer weiteren Ausführungsform erfolgt das Aufbringen der Wärme und der Kälte an derselben Stelle der Haut. Dazu ist in der Muschel ein Wärme/Kälteelement mit der Möglichkeit zur Flüssigkeitsaufnahme angeordnet. Über Schläuche wird dem Wärme/Kälteelement Heiz- bzw. Kühlflüssigkeit zugeführt, um es zu wärmen bzw. zu kühlen. Das Heizen und Kühlen des Wärme/Kälteelementes erfordert jedoch relativ viel Zeit, was einem schnellen Wechsel von warm zu kalt oder umgekehrt entgegensteht.

Eine weitere Vorrichtung zur Schmerzbehandlung mittels wechselweisem Zuführen von Wärme und Kälte ist aus der US 4,585,002 bekannt. Mittels Peltierelementen wird der zu behandelnden Körperzone im Wechsel Wärme und Kälte zugeführt. Die Temperaturen bewegen sich zwischen 25°C und 43°C. Eine Steuereinheit steuert dabei den Stromfluß zu den Peltierelementen derart, dass der Wechsel zwischen warm und kalt mehrmals pro Minute erfolgt.

Eine Vorrichtung zum Erzeugen einer Serie von Temperaturmustern zum Verringern von Schmerzen ist in EP 0 330 472 offenbart. Die Temperaturen werden mittels thermoelektrischer Elemente erzeugt und liegen im Bereich zwischen 19°C und 44°C. Ein Wechsel des Temperaturgradienten erfolgt höchstens einmal pro Minute.

Die US 5,746,702 offenbart eine Vorrichtung zur Hautmassage der Massageeffekt wird dadurch erreicht, dass der Haut im zyklischen Wechsel Wärme und Kälte zugeführt wird. Die Vorrichtung zum Durchführen der Hautmassage umfasst zwei Behandlungsblöcke, die mittels gebogener Federn an den Kopf der zu behandelnden Person angedrückt werden. Mittels Peltierelementen kann eine Kontaktplatte auf Temperaturen zwischen 25°C - 32°C erwärmt und auf Temperaturen zwischen 10°C - 17°C abgekühlt werden. Zum Erwärmen bzw. Abkühlen der Kontaktplatte versorgt ein Pulsgenerator die Peltierelemente mit bipolaren Strompulsen der Dauer von 60 - 90 Sekunden.

Gegenüber dem zitierten Stand der Technik ist es Aufgabe der vorliegenden Erfindung, eine Wärme/Kältevorrichtung zur Verfügung zu stellen, die sich in vorteilhafter Weise in einem Gerät für die Schmerzbehandlung einsetzen lässt.

US2002/0107543 A1 beschreibt eine physiotherapeutische Vorrichtung, die eine Temperatureinstelleinrichtung umfasst, welche in der Lage ist, einen Temperaturunterschied von mindestens 40°C innerhalb einer Minute zu realisieren. Die Temperatureinstellvorrichtung umfasst ein Wärmeübertragungsmedium welches über thermoelektrische Elemente geheizt wird. Das Wärmeübertragungsmedium wird an die zu behandelnde Körperzone angelegt. An die Seite des Wärmeübertragungsmediums, die der an die zu behandelnde Körperzone anzulegenden Seite gegenüber liegt, grenzt eine Kühleinheit zum Abführen überflüssiger Wärme an.

Gegenüber dem zitierten Stand der Technik ist es Aufgabe der vorliegenden Erfindung, eine Wärme/Kältevorrichtung zur Verfügung zu stellen, die sich in vorteilhafter Weise in einem Gerät für die Schmerzbehandlung einsetzen lässt.

Eine weitere Aufgabe der Erfindung ist es, ein Schmerzgerät mit einer gegenüber dem Stand der Technik verbesserten Wärme/Kältevorrichtung zur Verfügung zu stellen

Die erste Aufgabe wird durch eine Wärme/Kältevorrichtung nach Anspruch 1 gelöst, die zweite durch ein Schmerzbehandlungsgerät nach Anspruch 9. Die abhängigen Ansprüche beschreiben vorteilhafte Ausgestaltungen der Erfindung.

Eine erfindungsgemäße Wärme/Kältevorrichtung, insbesondere für ein Schmerzbehandlungsgerät, umfasst eine Heizeinrichtung und eine Kühleinrichtung zum wechselweisen Erwärmen und Kühlen eines Heiz/Kühlelementes auf eine hohe bzw. eine niedrige Temperatur. Die Heizeinrichtung und die Kühleinrichtung sind derart ausgestaltet und zueinander angeordnet, dass der Wechsel zwischen der hohen und der tiefen Temperatur innerhalb von drei Minuten möglich ist und die Temperaturdifferenz zwischen der hohen und der niedrigen Temperatur mindestens 40°C beträgt. Das Heiz/Kühlelement ist ein elektrische Heizplatte und weist eine Heiz/Kühlfläche auf, wobei die Heiz/Kühlfläche mindestens eine Oberfläche der Heizeinrichtung oder eine Oberfläche der Kühleinrichtung umfasst. Auf diese Weise ist ein unmittelbares Heizen bzw. Kühlen ohne Zwischenschaltung weiterer, den Heiz- bzw. Kühlvorgang verzögernder Elemente möglich. Insbesondere ist die Heiz/Kühlfläche von einer Oberfläche der Heizeinrichtung gebildet. Die Kühleinrichtung ist auf der der Heiz/Kühlfläche abgewandten Seite der Heizeinrichtung angeordnet. Außerdem weist die Heizeinrichtung eine geringe Wärmekapazität auf. Wenn während der Heizphase die Heizeinrichtung eingeschaltet ist, gibt Ihre Oberfläche Wärme an den zu behandelnden Körperbereich ab. Die Kühleinrichtung kann, muss aber nicht, während der Heizperiode ausgeschaltet sein. Während der Kühlphase, in der die Heizeinrichtung aus- und die Kühleinrichtung eingeschaltet ist, verliert die Heizeinrichtung aufgrund ihrer geringen Wärmekapazität rasch ihre Restwärme, so dass ihre Oberfläche vom Kühlelement rasch auf die niedrige Temperatur abgekühlt werden kann.

Die geringe Wärmekapazität der elektrischen Heizplatte kann bspw. dadurch erreicht werden, dass die Heizplatte weniger als 0,5 mm dick ausgestaltet wird. In einer besonders vorteilhaften Ausgestaltung beträgt die Dicke der Heizplatte ca. 0,1 mm. Anstatt der oder zusätzlich zur geeigneten Wahl der Dicke der Heizplatte kann eine geringe Wärmekapazität auch durch geeignete Wahl des verwendeten Materials erzielt werden.

Die Heiz- und die Kühleinrichtung können hierbei als Wärme bzw. Kälte erzeugende Einrichtungen oder als Wärme bzw. Kälte übertragende Einrichtungen ausgestaltet sein.

Der Erfindung liegt die Einsicht zugrunde, dass sich bei einer Schmerzbehandlung, insbesondere bei einer Migränebehandlung, gute Behandlungserfolge erzielen lassen, wenn dem Patienten Temperaturunterschiede von mindestens 40°C in relativ geringen Zeitabständen, insbesondere in Zeitabständen von weniger als 3 Minuten, vermittelt werden. Mit den aus dem Stand der Technik bekannten Wärme/Kältevorrichtungen lassen sich derartige Temperaturdifferenzen in so kurzen Zeiträumen nicht realisieren .

Vorteilhafterweise liegt die hohe Temperatur bei mindestens 50°C und die niedrige Temperatur bei höchstens +10°C. Besonders vorteilhaft ist es, wenn die hohe Temperatur zwischen 50°C und 60°C, insbesondere bei 60°C, und die tiefe Temperatur zwischen -20°C und 0°C, insbesondere bei -20°C, liegt. Mit der vorliegenden Erfindung ist es insbesondere möglich, innerhalb von weniger als einer Minute, insbesondere in nur 30 Sekunden Temeperaturdifferenzen von bis zu 80°C zu erzeugen, d.h. die Behandlungstemperatur um 80°C zu verändern, was zu besonders guten Behandlungserfolgen führt. Zudem ermöglicht die erfindungsgemäße Wärme/Kältevorrichtung das Aufbringen der Wärme und der Kälte auf dieselbe Behandlungsstelle.

Als Kühleinrichtung kann ein thermoelektrisches Element, insbesondere ein Peltierelement, Verwendung finden. Zum Abführen überschüssiger Wärme kann außerdem an der der Heizeinrichtung abgewandten Seite des thermoelektrischen Elementes ein Kühlkörper angeordnet sein.

Ein erfindungsgemäßes Schmerzbehandlungsgerät ist mit einer erfindungsgemäßen Wärme/Kältevorrichtung ausgestattet. Ein derartig ausgestaltetes Schmerzbehandlungsgerät ermöglicht ein wirksames Behandeln von Schmerzen, insbesondere von Migräne.

Weitere Merkmale, Eigenschaften und Vorteile der vorliegenden Erfindung werden nachfolgend anhand eines Ausführungsbeispiels unter Bezugnahme auf die beiliegende Figur beschrieben.

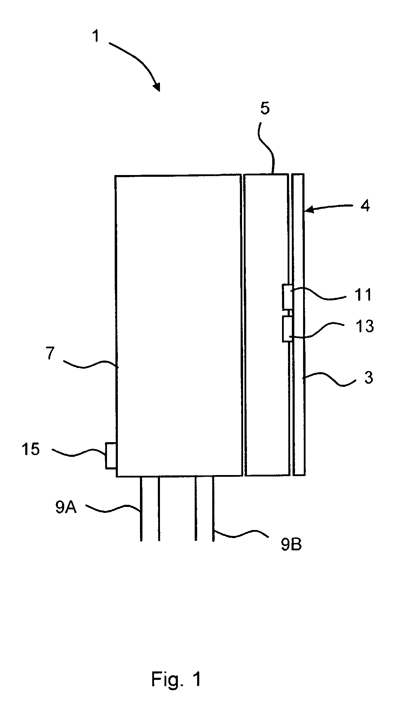

- Fig. 1: zeigt ein erstes Ausführungsbeispiel für die erfindungsgemäße Wärme/Kältevorrichtung.

Ein Ausführungsbeispiel für die vorliegende Erfindung ist in Fig. 1 dargestellt. Das in Figur 1 gezeigte Heiz/Kühlelement 1, welches als Heiz/Kühlelement in einem Schmerzbehandlungsgerät Verwendung findet, umfasst eine elektrische Heizplatte 3 als Heizeinrichtung und ein Peltierelement 5 als Kühleinrichtung. Es kann in eine Muschel integriert sein, die in Art eines Kopfhörers am Ende eines auf dem Kopf zu tragenden Bügels befestigt ist. Vorteilhafterweise umfasst der Bügel zwei derartige Muscheln, je eine an jedem Ende des Bügels. Alternativ kann das Heiz/Kühlelement 1 auch an einem Band befestigt sein, welches am Körper zu tragen ist. Beispiele für die Ausgestaltung einer derartigen Muschel bzw. eines derartigen Bandes sind bspw. in DE 100 65 592 beschrieben.

Statt das Heiz/Kühlelement 1 in eine an einem Bügel befestigte Muschel zu integrieren, kann es aber auch direkt am Bügel befestigt werden. Eine derartige Ausgestaltung ist bspw. der US 5,746,702 zu entnehmen.

Unabhängig davon, ob das Heiz/Kühlelement 1 in einer Muschel angeordnet oder direkt am Bügel befestigt ist, wird der Bügel dem Patienten bei der Behandlung vorzugsweise so aufgesetzt, dass die an den Enden des Bügels angeordneten Heiz/Kühlelemente 1 an den Schläfen anliegen.

Während der Behandlung erfolgt im Wechsel ein Heizen des Heiz/Kühlelements 1 auf Werte zwischen 50°C und 60°C, insbesondere auf 60°C, und ein Kühlen des Heiz/Kühlelements 1 auf Werte zwischen -20°C und 0°C, insbesondere auf -20°C. Dabei werden die Heizplatte 3 und das Peltierelement 5 im Wechsel derart mit elektrischer Energie versorgt, dass der Temperaturwechsel von +60°C auf -20°C innerhalb von 30 Sekunden erfolgt. Der derart rasche Temperaturwechsel wird durch einen speziellen Aufbau das Heiz/Kühlelements 1 ermöglicht.

Die Oberfläche 4 der elektrischen Heizplatte 3 dient zum Applizieren der Wärme bzw. Kälte auf die Haut des Patienten. Sie kann mit einem elektrisch isolierenden Überzug, bspw. einem Kunststoffüberzug, versehen sein. Dieser sollte jedoch eine hohe thermische Leitfähigkeit aufweisen, um die Applikation der Wärme bzw. Kälte nicht negativ zu beeinflussen.

Die elektrische Heizplatte besitzt eine Dicke von 0,1 mm sowie eine Fläche von 40 x 40 mm und ist aus einer Kupferlegierung, bspw. FX 9, hergestellt. Aufgrund ihrer geringen Dicke fällt die Wärmekapazität der Heizplatte 3 entsprechend gering aus, was ihr Auskühlen nach dem Ende einer Heizphase beschleunigt. Sie hat einen kleinen Leitwert von ca. 2 MS/m und wird während der Heizphase von einem Strom der Stärke 30 A bis 70 A durchflossen. Im vorliegenden Ausführungsbeispiel betragt die Stromstärke 65 A und die angelegte Spannung 0,5 V.

Mittels eines wärmeleitfähigen Klebers ist die elektrische Heizplatte 3 auf das Peltierelement 5 aufgeklebt. Mit dem Peltierelement 5 wird während der Kühlphase die Kälteleistung erzeugt. Aufgrund der geringen Dicke der elektrischen Heizplatte 1 steht die Kälteleistung sehr schnell zur Verfügung. Das Peltierelement 5 erzeugt bei Stromfluss (im vorliegenden Ausführungsbeispiel 5 A bei 16 V) auf einer Seite Kälte, während im Gegenzug auf der anderen Seite des Elementes Wärme entsteht. Um diese Wärme rasch abzuführen, ist auf seiner der elektrischen Heizplatte 3 abgewandten Seite ein Wasserkühlkörper 7 angeordnet. Dieser ist an einen geschlossenen Wasserkreislauf angeschlossen, über den die überschüssige Wärme abgeführt wird. Vom Wasserkreislauf sind in Fig. 1 die Zuführleitung 9A sowie die Abführleitung 9B dargestellt. Statt einer Wasserkühlung kann auch eine Luftkühlung erfolgen, sofern die abzuführende Wärmemenge dies zuläßt. In diesem Fall weist der Kühlkörper statt eines Anschlusses an einen geschlossenen Wasserkreislauf Kühlrippen für die Luftkühlung auf.

Drei Temperaturfühler 11, 13, 15 überwachen während des Betriebs des Heiz/Kühlelements 1 den Temperaturverlauf. Der Temperaturfühler 11 gibt sein Temperatursignal an einen Steuerkreis aus, während der Temperaturfühler 13, der die maximalen Temperaturwerte auf der Heizplatte 3 überwacht, und der Temperaturfühler 15, der die Temperatur des Kühlkörpers 7 überwacht, ihre Temperatursignale an einen Sicherheitskreis ausgeben. Bei Überschreiten vorgegebener Sicherheitswerte für die entsprechenden Temperaturen schaltet der Sicherheitskreis das Gerät auf Störung und beendet den Betrieb.

## Patentansprüche

1. Wärme/Kältevorrichtung für ein Schmerzbehandlungsgerät, mit einer Heizeinrichtung (3) und einer Kühleinrichtung (5) zum wechselseitigen Erwärmen und Kühlen eines Heiz/Kühlelementes (1) auf eine hohe bzw. niedrige Temperatur, wobei die Heizeinrichtung (3) und die Kühleinrichtung (5) derart ausgestaltet und zueinander angeordnet sind, dass der Wechsel zwischen der hohen und der tiefen Temperatur innerhalb von drei Minuten möglich ist, wobei die Temperaturdifferenz zwischen der hohen und der niedrigen Temperatur mindestens 40°C beträgt **dadurch gekennzeichnet, dass** die Heizeinrichtung eine elektrische Heizplatte (3) ist, eine Oberfläche (4) der elektrischen Heizplatte (3) die Heiz/Kühlfläche bildet, die Kühleinrichtung (5) auf der der Heiz/Kühlfläche abgewandten Seite der elektrischen Heizplatte (3) angeordnet ist und die elektrischen Heizplatte (3) eine geringe Wärmekapazität aufweist.

2. Wärme/Kältevorrichtung nach Anspruch 1, **dadurch gekennzeichnet, dass** die Dicke der Heizplatte (3) weniger als 0,5 mm beträgt.

3. Wärme/Kältevorrichtung nach einem der Ansprüche 1 oder 2, **dadurch gekennzeichnet, dass** die hohe Temperatur mindestens 50°C und die tiefe Temperatur höchstens +10°C beträgt.

4. Wärme/Kältevorrichtung nach Anspruch 3, **dadurch gekennzeichnet, dass** die tiefe Temperatur weniger als 0°C beträgt.

5. Wärme/Kältevorrichtung nach einem der Ansprüche 1 bis 4, **dadurch gekennzeichnet, dass** die Heizplatte (3) und die Kühleinrichtung (5) derart ausgestaltet und zueinander angeordnet sind, dass ein Wechsel zwischen der hohen und der tiefen Temperatur innerhalb von einer Minute möglich ist.

6. Wärme/Kältevorrichtung nach Anspruch 5, **dadurch gekennzeichnet, dass** die Heizplatte (3) und die Kühleinrichtung (5) derart ausgestaltet und zueinander angeordnet sind, dass ein Wechsel zwischen der hohen und der tiefen Temperatur innerhalb von 30 Sekunden möglich ist.

7. Wärme/Kältevorrichtung nach einem der Ansprüche 1 bis 6, **dadurch gekennzeichnet, dass** die Kühleinrichtung ein thermoelektrisches Element (5) ist.

8. Wärme/Kältevorrichtung nach einem der Ansprüche 1 bis 7, **dadurch gekennzeichnet, dass** an der der Heizplatte (3) abgewandten Seite des thermoelektrischen Elementes (5) ein Kühlkörper (7) zum Abführen überschüssiger Wärme angeordnet ist.

9. Schmerzbehandlungsgerät mit einer Wärme/Kältevorrichtung nach einem der Ansprüche 1 bis 8.

## Claims

1. A warming/cooling apparatus for a pain treatment unit, comprising a heating device (3) and a cooling device (5) for alternately heating and cooling a heating/cooling element (1) to a high and a low temperature respectively, wherein the heating device (3) and the cooling device (5) are adapted and arranged relative to each other in such a way that the change between the high and the low temperatures is possible within three minutes, wherein the temperature difference between the high and the low temperatures is at least 40° C, **characterised in that** the heating device is an electrical heating plate (3), a surface (4) of the electrical heating plate (3) forms the heating/cooling face, the cooling device (5) is arranged on the side of the electrical heating plate (3), that is remote from the heating/cooling face, and the electrical heating device (3) has a low heat capacity.

2. A warming/cooling apparatus according to claim 1 **characterised in that** the thickness of the heating plate (3) is less than 0.5 mm.

3. A warming/cooling apparatus according to one of claims 1 and 2 **characterised in that** the high temperature is at least 50°C and the low temperature is at most +10°C.

4. A warming/cooling apparatus according to claim 3 **characterised in that** the low temperature is less than 0°C.

5. A warming/cooling apparatus according to one of claims 1 to 4 **characterised in that** the heating plate (3) and the cooling device (5) are adapted and arranged relative to each other in such a way that a change between the high and the low temperatures is possible within a minute.

6. A warming/cooling apparatus according to claim 5 **characterised in that** the heating plate (3) and the cooling device (5) are adapted and arranged relative to each other in such a way that a change between the high and the low temperatures is possible within 30 seconds.

7. A warming/cooling apparatus according to one of claims 1 to 6 **characterised in that** the cooling device is a thermoelectric element (5).

8. A warming/cooling apparatus according to one of claims 1 to 7 **characterised in that** a cooling body (7) for dissipating excess heat is arranged at the side of the thermoelectric element (5), that is remote from the heating plate (3).

9. A pain treatment unit comprising a warming/cooling apparatus according to one of claims 1 to 8.

## Revendications

1. Dispositif de chauffage/refroidissement pour appareil de traitement de la douleur, comprenant un dispositif de chauffage (3) et un dispositif de refroidissement (5) destinés au chauffage et au refroidissement alternés d'un élément chauffant/de refroidissement (1) à une température élevée ou basse, le dispositif de chauffage (3) et le dispositif de refroidissement (5) étant configurés et disposés l'un par rapport à l'autre de telle sorte que le passage de la haute température à la basse température puisse s'effectuer en l'espace de trois minutes, la différence de température entre la haute température et la basse température étant d'au moins 40°C, **caractérisé en ce que** le dispositif de chauffage est une plaque chauffante électrique (3), qu'une surface (4) de la plaque chauffante électrique (3) forme la surface de chauffage/refroidissement, que le dispositif de refroidissement (5) est disposé sur le côté de la plaque chauffante électrique (3) opposé à la surface de chauffage/refroidissement et que la plaque chauffante électrique (3) présente une faible capacité calorifique.

2. Dispositif de chauffage/refroidissement selon la revendication 1, **caractérisé en ce que** l'épaisseur de la plaque chauffante (3) est inférieure à 0,5 mm.

3. Dispositif de chauffage/refroidissement selon l'une des revendications 1 ou 2, **caractérisé en ce que** la haute température est d'au moins 50 °C et que la basse température n'excède pas +10 °C.

4. Dispositif de chauffage/refroidissement selon la revendication 3, **caractérisé en ce que** la basse température est inférieure à 0 °C.

5. Dispositif de chauffage/refroidissement selon l'une des revendications 1 à 4, **caractérisé en ce que** la plaque chauffante (3) et le dispositif de refroidissement (5) sont configurés et disposés l'un par rapport à l'autre de telle sorte qu'un passage de la haute température à la basse température puisse s'effectuer en l'espace d'une minute.

6. Dispositif de chauffage/refroidissement selon la revendication 5, **caractérisé en ce que** la plaque chauffante (3) et le dispositif de refroidissement (5) sont configurés et disposés l'un par rapport à l'autre de telle sorte qu'un passage de la haute température à la basse température puisse s'effectuer en l'espace de 30 secondes.

7. Dispositif de chauffage/refroidissement selon l'une des revendications 1 à 6, **caractérisé en ce que** le dispositif de refroidissement est un élément thermoélectrique (5).

8. Dispositif de chauffage/refroidissement selon l'une des revendications 1 à 7, **caractérisé en ce que** sur le côté de l'élément thermoélectrique (5) opposé à la plaque chauffante (3) est disposé un dissipateur thermique (7) destiné à évacuer l'excédent de chaleur.

9. Appareil de traitement de la douleur comprenant un dispositif de chauffage/refroidissement selon l'une des revendications 1 à 8.
